(19) Europäisches Patentamt
European Patent Office
Office européen des brevets

(11) Veröffentlichungsnummer: **0 456 067 A1**

# (12) EUROPÄISCHE PATENTANMELDUNG

(21) Anmeldenummer: **91106891.4**

(51) Int. Cl.[5]: **C07D 241/44**, A01N 43/60

(22) Anmeldetag: **27.04.91**

(30) Priorität: **10.05.90 DE 4014937**

(43) Veröffentlichungstag der Anmeldung:
**13.11.91 Patentblatt 91/46**

(84) Benannte Vertragsstaaten:
**AT BE CH DE DK ES FR GB GR IT LI LU NL SE**

(71) Anmelder: **BAYER AG**
**W-5090 Leverkusen 1 Bayerwerk(DE)**

(72) Erfinder: **de Jong, Anno, Dr.**
**Ursulaweg 7**
**W-4018 Langenfeld(DE)**
Erfinder: **Fuchs, Rainer, Dr.**
**Am Rohm 107**
**W-5600 Wuppertal 1(DE)**

## (54) 2,3-Disubstituierte Chinoxaline als Leistungsförderer für Tiere.

(57) Die vorliegende Erfindung betrifft die Verwendung von 2,3-disubstituierten Chinoxalinen der Formel (I)

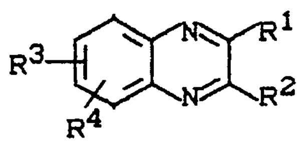

(I)

in welcher

$R^1$ für Halogen, OH, SH, Alkoxy, Aryloxy, Alkylthio, Arylthio steht,

$R^2$ für Halogen, OH, SH, Alkoxy, Aryloxy, Alkylthio, Arylthio steht,
$R^1$ und $R^2$ gemeinsam für den Rest der Formel

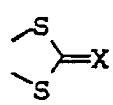

stehen, in welcher X für O oder S steht,

$R^3$ für Wasserstoff, Halogen, CN oder die Reste

$-CONR^5R^6$, $-SO_2NR^5R^6$, $-SO_2OR^5$,
CN
|
$-CH-R^7$, $-O-R^7$, $-COOR^5$ steht,

$R^4$ für H, Alkyl, Halogen, $NO_2$ oder den Rest $-CONR^5R^6$ steht,

$R^5$ für H, Alkyl oder Cycloalkyl steht, die gegebenenfalls substituiert sind oder für Aryl steht, das gegebenenfalls substituiert ist,

EP 0 456 067 A1

$R^6$ für H, Alkyl oder Cycloalkyl steht, die gegebenenfalls substituiert sind, oder für Aryl steht, das gegebenenfalls substituiert ist oder für den Rest -S-$R^7$ steht,

$R^5$ und $R^7$ können gemeinsam mit dem angrenzenden N-Atom einen gegebenenfalls substituierten heterocyclischen Ring bilden, der gegebenenfalls N-oder O- als weitere Heteroatome enthalten kann;

$R^7$ für gegebenenfalls substituiertes Alkyl oder Aryl steht,

zur Leistungsförderung von Tieren, insbesondere zur Beeinflussung der Pansenfermentation von Wiederkäuern.

Die vorliegende Erfindung betrifft die Verwendung von 2,3-disubstituierten Chinoxalinen als Leistungsförderer für Tiere, insbesondere als Mittel zur Beeinflussung der Pansenfermentation von Wiederkäuern, sowie neue 2,3-disubstituierte Chinoxaline und ihre Herstellung.

2,3-disubstituierte Chinoxaline und ihre Wirkung als Fungizide und Bakterizide für die Landwirtschaft sind schon bekannt (EP-OS 20 119; GB-P 1 043 042, DAS 1 100 372).

2,3-disubstituierte Chinoxaline und ihre Wirkung als Mittel zur Inhibierung der Sekretion von Magensäure sind schon bekannt (GB-P 1 178 930; US-P 3 691 166, 3 510 487).

Fermentativ hergestellte ionophore Polyether sowie davon abgeleitete synthetisch hergestellte Kronenether und ihre Wirkung zur Beeinflussung der Pansenfermentation von Wiederkäuern sind schon bekannt (J. Med. Chem. 1990 (23), S. 765 bis 771; US-P 4 777 270).

Bis auf die erwähnten Kronenether sind noch keine vollsynthetisch hergestellten Verbindungen bekannt bei denen sich in-vitro Hinweise auf eine Beeinflussung der Pansenfermentation von Wiederkäuern auch in in-vivo Versuchen zufriedenstellend bestätigt haben (J. Med. Chem. 1990 (23), S. 765 bis 771; US-P 4 800 213; US-P 4 801 618).

Chinoxalin-di-N-oxide und ihre Verwendung als Fütterungsantibiotika und Leistungsförderer für Nutztiere insbesondere für Schweine und junge Kälber sind bekannt. In in-vitro Versuchen zeigen diese Verbindungen jedoch eine negative Beeinflussung der Pansenfermentation. Sie eignen sich daher nicht als Mittel zur Beeinflussung der Pansenfermentation von Wiederkäuern.

Gegenstand der vorliegenden Erfindung sind daher:

1. Verwendung von 2,3-disubstituierten Chinoxalinen der Formel (I)

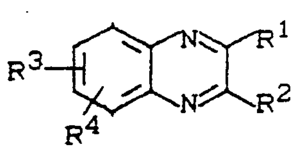

(I)

in welcher

| | |
|---|---|
| $R^1$ | für Halogen, OH, SH, Alkoxy, Aryloxy, Alkylthio, Arylthio steht, |
| $R^2$ | für Halogen, OH, SH, Alkoxy, Aryloxy, Alkylthio, Arylthio steht, |
| $R^1$ und $R^2$ | gemeinsam für den Rest der Formel |

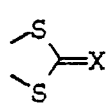

stehen, in welcher X für O oder S steht,

$R^3$ für H, Halogen, CN oder die Reste $-CONR^5R^6$,

$-SO_2NR^5R^6$, $-SO_2OR^5$,
CN
|
$-CH-R^7$, $-O-R^7$, $-COOR^5$ steht,

| | |
|---|---|
| $R^4$ | für H, Alkyl, Halogen, $NO_2$ oder den Rest $-CONR^5R^6$ steht, |
| $R^5$ | für H, Alkyl oder Cycloalkyl steht, die gegebenenfalls substituiert sind oder für Aryl steht das gegebenenfalls substituiert ist, |
| $R^6$ | für H, Alkyl oder Cycloalkyl steht, die gegebenenfalls substituiert sind oder für Aryl steht das gegebenenfalls substituiert ist oder für den Rest $-S-R^7$ steht, |
| $R^5$ und $R^7$ | können gemeinsam mit dem angrenzenden N-Atom einen gegebenenfalls substituierten heterocyclischen Ring bilden, der gegebenenfalls N- oder O- als weitere Heteroatome enthalten kann; |
| $R^7$ | für gegebenenfalls substituiertes Alkyl oder Aryl steht, |

zur Leistungsförderung von Tieren, insbesondere zur Beeinflussung der Pansenfermentation von Wiederkäuern.

2. Neue 2,3-disubstituierte Chinoxaline der Formel (II)

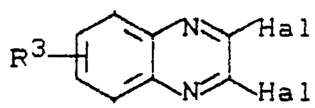

(II)

in welcher

$R^3$ für die Reste $-CONR^5R^6$ oder $-SO_2NR^5R^6$ steht,

$R^6$ für H, Alkyl steht,

$R^5$ für Alkyl steht das durch Fluor substituiert ist,

Hal für Chlor oder Brom steht.

3. Verfahren zur Herstellung der neuen 2,3-disubstituierten Chinoxaline der Formel (II)

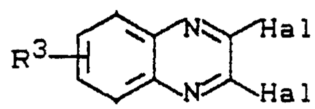

(II)

in welcher

$R^3$ für die Reste $-CONR^5R^6$ oder $-SO_2NR^5R^6$ steht,

$R^6$ für H, Alkyl steht,

$R^5$ für Alkyl steht das durch Fluor substituiert ist,

Hal für Chlor oder Brom steht,

dadurch gekennzeichnet, daß man 2,3-disubstituierte Chinoxaline der Formel (III)

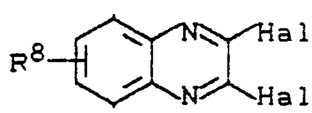

(III)

in welcher

$R^8$ für die Reste

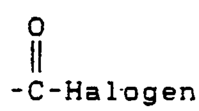

oder $-SO_2$-Halogen steht und

Hal für Chlor oder Brom steht,

mit Aminen der Formel (IV)

$H-NR^5R^6$ (IV)

in welcher

$R^5$ und $R^6$ die oben angegebene Bedeutung haben,

umsetzt.

Die erfindungsgemäß verwendbaren Verbindungen der Formel (I) zeigen sowohl in in-vitro als auch in in-vivo Versuchen gute Wirkung bei der Beeinflussung der Pansenfermentation von Wiederkäuern.

Bevorzugt werden Verbindungen der Formel (I) eingesetzt, in welcher

$R^1$ für Brom, Chlor, OH, SH, $C_{1-6}$-Alkoxy, gegebenenfalls substituiertes Phenoxy, $C_{1-6}$-Alkylthio, gegebenenfalls substituiertes Phenylthio steht,

$R^2$ für die bei $R^1$ angegebenen Reste steht,
$R^1$ und $R^2$ gemeinsam für den Rest der Formel

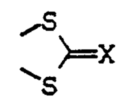

stehen, in welcher X für O oder S steht,
$R^3$ für H, Halogen, CN oder die Reste $-CONR^5R^6$,

$$-SO_2NR^5R^6,\ -SO_2OR^5,\ -\underset{\displaystyle |}{\overset{CN}{}}\!\!CH-R^7,\ -O-R^7,\ -COOR^5 \text{ steht,}$$

$R^4$ für H, $C_{1-6}$-Alkyl, Fluor, Chlor, Brom, $NO_2$ oder den Rest $-CONR^5R^6$ steht,
$R^5$ für H, $C_{1-6}$-Alkyl, $C_{3-6}$-Cycloalkyl steht, die gegebenenfalls substituiert sind, Phenyl das gegebenenfalls substituiert ist steht,
$R^6$ für die bei $R^5$ angegebenen Reste oder $-S-R^7$ steht,
$R^5$ und $R^6$ gemeinsam mit dem angrenzenden Stickstoffatom für einen 5- oder 6-gliedrigen gesättigten oder ungesättigten heterocyclischen Ring stehen der als zusätzliche Heteroatome N oder O enthalten kann und gegebenenfalls substituiert ist,
$R^7$ für gegebenenfalls substituiertes $C_{1-6}$-Alkyl oder Phenyl steht.

Die gegebenenfalls substituierten Reste können einen oder mehrere, vorzugsweise 1 bis 3, insbesondere 1 oder 2 gleiche oder verschiedene Substituenten tragen. Als Substituenten seien beispielhaft und vorzugsweise aufgeführt; Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio, Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere fluor stehen, wie Trifluormethyl; Halogenalkoxy mit vorzugsweise 1 bis 4, insbesondere 1 bis 2 Kohlenstoffatomen und vorzugsweise 1 bis 7, insbesondere 1 bis 6 Halogenatomen, wobei die Halogenatome gleich oder verschieden sind und als Halogenatome vorzugsweise Fluor, Chlor oder Brom, vorzugsweise Fluor oder Chlor stehen, wie Trifluormethoxy, Hexafluor-n-propoxy, Fluorchlor-ethoxy; Halogenalkylthio mit vorzugsweise 1 bis 4, insbesondere 1 bis 2 Kohlenstoffatomen und vorzugsweise 1 bis 7, insbesondere 1 bis 6 Halogenatomen, wobei die Halogenatome gleich oder verschieden sind und als Halogenatoome vorzugsweise Fluor, Chlor oder Brom, vorzugsweise Fluor oder Chlor stehen, Hydroxy; Halogen, vorzugsweise fluor, Chlor, Brom und Iod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy;
Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl.

Besonders bevorzugt werden Verbindungen der Formel (I) eingesetzt, in welcher
$R^1$ für Brom oder Chlor steht,
$R^2$ für Brom oder Chlor steht,
$R^1$ und $R^2$ gemeinsam für den Rest

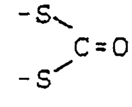

stehen,

$R^3$ für die Reste $-CONR^5R^6$ oder $-SO_2-NR^5R^6$ steht,

$R^4$ für H, $C_{1-6}$-Alkyl, Fluor, Chlor, Brom oder den Rest $-CONR^5R^6$ steht,

$R^5$ für H, $C_{1-6}$-Alkyl das gegebenenfalls substituiert ist durch Halogenatome, insbesondere Fluor oder Chlor, $C_{1-4}$-Alkoxy insbesondere Methoxy oder Ethoxy, $C_{1-4}$-Halogenalkoxy mit 1 bis 6 Halogenatomen, insbesondere Fluor, Phenyl oder Phenoxy die gegebenenfalls durch die obengenannten Reste substituiert sind, weiter steht $R^5$ für Phenyl das gegebenenfalls substituiert ist durch Alkyl mit 1 bis 4 Kohlenstoffatomen, wie Methyl, Ethyl, n- und i-Propyl und n-, i- und t-Butyl; Alkoxy mit 1 bis 4 Kohlenstoffatomen, wie Methoxy, Ethoxy, n- und i-Propyloxy und n-, i- und t-Butyloxy die gegebenenfalls durch 1 bis 6 Halogenatome, insbesondere Fluor oder Chlor substituiert sein können wie Trifluormethoxy, Fluorchlorethoxy, Hexafluorpropoxy; Alkylthio mit 1 bis 4 Kohlenstoffatomen, wie Methylthio, Ethylthio, n-und i-Propylthio und n-, i- und t-Butylthio; Halogenalkyl mit 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl;

$R^6$ für die bei $R^5$ angegebenen Reste stehen,

die Reste $R^5$ und $R^6$ gemeinsam mit dem angrenzenden N-Atom einen 5 oder 6 gliedrigen Heteroring bilden.

Ganz besonders bevorzugt sind Verbindungen der Formel (I), in welcher

$R^1$ und $R^2$ für Chlor stehen,

$R^3$ für die Reste $-CONR^5R^6$ oder $-SO_2NR^5R^6$ steht,

$R^4$ für Wasserstoff steht,

$R^5$ für $C_{1-4}$-Alkyl steht, das gegebenenfalls durch 1 bis 3 Halogenatome substituiert ist, ferner für Phenyl steht, das durch $C_{1-4}$-Alkyl, Halogenalkyl mit 1 bis 4 C-Atomen und 1 bis 6 Halogenatomen, Halogenalkoxy mit 1 bis 4 C-Atomen und 1 bis 6 Halogenatomen substituiert ist,

$R^6$ für Wassserstoff oder $C_{1-4}$-Alkyl steht.

Im einzelnen seien folgende Verbindungen der Formel (I) genannt:

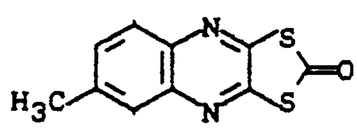
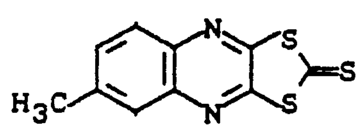
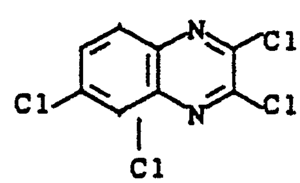

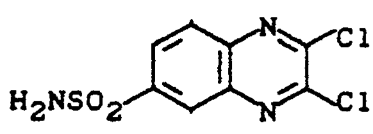
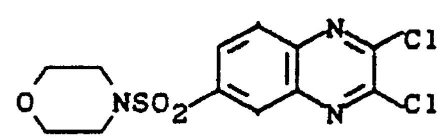

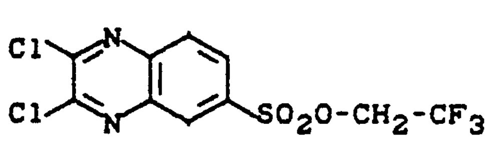
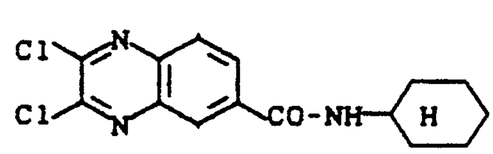

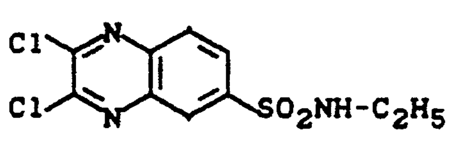
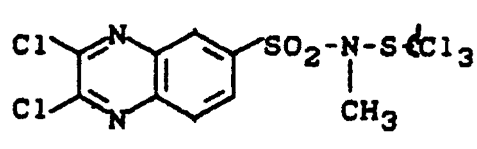

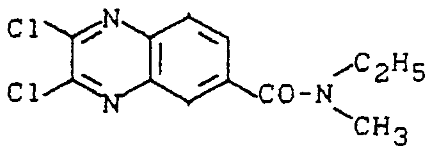
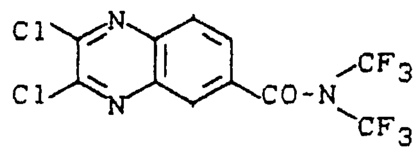

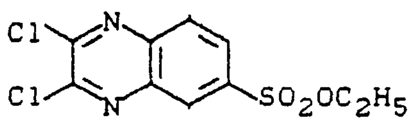
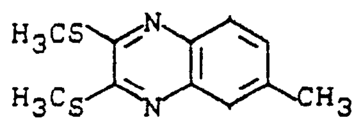

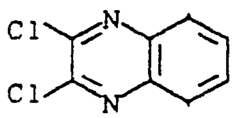
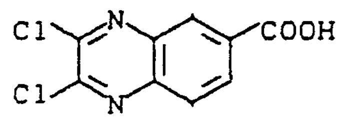

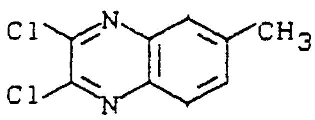
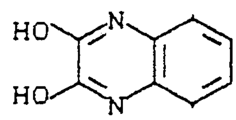

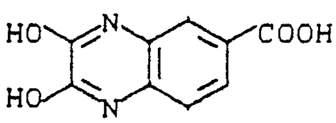
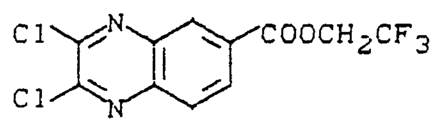

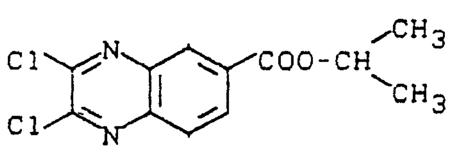
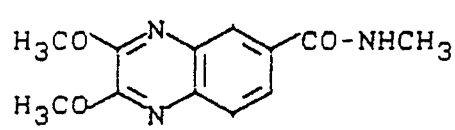

Bei den neuen 2,3-disubstituierten chinoxalinen Verbindungen der Formel (II) sind solche bevorzugt, in welcher

$R^5$ für $C_{1-4}$-Alkyl mit 1 bis 6 Fluoratomen, insbesondere Trifluormethyl, Trifluorpropyl steht,

$R^6$ für Wasserstoff steht und

Hal für Chlor steht.

Die neuen Verbindungen der Formel (II) werden hergestellt, indem man Verbindungen der Formel (III) mit Aminen der Formel (IV) in Gegenwart von Verdünnungsmitteln sowie gegebenenfalls in Gegenwart von Säureakzeptoren umsetzt.

Die Verbindungen der Formeln (III) und (IV) sind bekannt.

Das Verfahren wird durchgeführt, indem man in einer Mischung der Verbindung der Formel (IV) und einem Säureakzeptor in einem Verdünnungsmittel eine äquivalente Menge der Verbindung der Formel (III) zugibt. Die Reaktion wird bei Normaldruck zwischen 15 und 150° C, bevorzugt bei Siedetemperatur des Verdünnungsmittels durchgeführt.

Als Säureakzeptoren kommen in Frage Alkalicarbonate, tertiäre aliphatische oder aromatische Amine z.B. Trimethylamin, Triethylamin, Dimethylbenzylamin, Pyridin, 4-Dimethylaminopyridin.

Als Verdünnungsmittel kommen in Frage gegebenenfalls halogenierte aliphatische und aromatische Kohlenwasserstoffe wie Pentan, Hexan, Benzol, Toluol, Methylenchlorid, Chloroform, Chlorbenzol; Ether wie

Diethylether, Tetrahydrofuran, Dioxan; Nitrile wie Acetonitril, Benzonitril.

Die Wirkstoffe eignen sich bei günstiger Warmblütertoxizität als Mittel zur Leistungsförderung bei Zucht- und Nutztieren. Sie dienen dabei zur Förderung und Beschleunigung des Wachstums, der Milch- und Wollproduktion, sowie zur Verbesserung der Futterverwertung.

Zu den Nutz- und Zuchttieren gehören Wiederkäuer wie z.B. Rinder, Schafe, Ziegen sowie monogastrische Tiere die eine fermentative Gärung im Verdauungstrakt besitzen wie z.B. Schweine, Pferde, Geflügel, Kaninchen.

Die Wirkstoffe werden unabhängig vom Geschlecht der Tiere während allen Leistungsphasen der Tiere eingesetzt. Bevorzugt werden die Wirkstoffe während der intensiven Leistungsphase eingesetzt. Die intensive Leistungsphase dauert je nach Tierart von einem Monat bis zu 10 Jahren. Besonders wertvoll erweisen sich die Wirkstoffe bei Aufzucht und Haltung von Jung- und Masttieren.

Die Anwendung der Wirkstoffe erfolgt direkt oder in Form von für Tiere geeigneten Zubereitungen enteral. Die enterale Anwendung der Wirkstoffe geschieht z.B. oral in Form von Pulvern, Tabletten, Kapseln, Pasten, Tränken, Granulaten, Boli, über oral applizierbare Lösungen, Emulsionen oder Suspensionen sowie über das Futter oder über das Trinkwasser.

Besonders hervorgehoben seien Zubereitungen zur Verabreichung über das Futter oder das Trinkwasser. Dabei können die Wirkstoffe dem Futter direkt oder in Form von Prämixen oder Futterkonzentraten zugesetzt werden.

Zum Futter zählen Einzelfuttermittel pflanzlicher Herkunft wie Heu, Rüben, Getreide und Getreidenebenprodukte, Melasse, Silage, Einzelfuttermittel tierischer Herkunft wie Fleisch, Fette, Milchprodukte, Knochenmehl, Fischprodukte, die Einzelfuttermittel wie Vitamine, Proteine, Zucker, Stärke, Mehle, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie- und Proteinversorgung sowie der Versorgung mit Vitaminen, Mineralsalzen und Spurenelementen sicherstellt.

Prämixe und Futterkonzentrate sind Mischungen des Wirkstoffs mit Trägerstoffen und gegebenenfalls weiteren Hilfsstoffen. Zu den Trägerstoffen zählen alle Einzelfuttermittel oder Gemische derselben.

Die Wirkstoffe können in den Zubereitungen allein oder in Mischung mit anderen leistungsfördernden Wirkstoffen, mineralischen Futtermitteln, Spurenelement-Verbindungen, Vitaminen, stickstoffhaltigen nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind z.B. Antibiotika wie Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid, Selenverbindungen.

Vitamine sind z.B. Vitamin A, Vitamin $D_3$, Vitamin E.

Stickstoffhaltige nicht-Protein-Verbindungen sind z.B. Biuret, Harnstoff.

Farbstoffe sind z.B. Carotinoide wie Canthaxidin, Zeaxanthin, Capsanthin oder Farbstoffe, die zur Färbung von Lebensmitteln zugelassen sind.

Antioxidantien sind z.B. Ethoxyquin, Butylhydroxy-Toluol, Ascorbinsäure.

Aromastoffe sind z.B. Vanillin.

Emulgatoren sind z.B. Ester der Milchsäure, Lecithin. Fließhilfsstoffe sind z.B. Natriumstearat, Calciumstearat, Kieselsäuren, Bentonite, Ligninsulfonate.

Konservierungsstoffe sind z.B. Propionsäure, Calciumpropionat, Sorbinsäure, Ascorbinsäure.

Preßhilfsstoffe sind z.B. Ligninsulfonate, Celluloseether.

Die Konzentration der Wirkstoffe im Futter beträgt normalerweise etwa 0,01-5000 ppm, bevorzugt 10-1000 ppm.

Die Konzentration der Wirkstoffe in den Prämixen oder Futterkonzentraten beträgt ca. 0,5 bis 50 Gewichtsprozent, bevorzugt 1 bis 20 Gewichtsprozent.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 500 mg/kg insbesondere 0,1 bis 50 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung der unten angegebenen Zusammensetzung und 2,5 g Wirkstoff-Prämix der unten angegebenen Zusammensetzung ergeben nach sorgfältigem Mischen 1 kg Futter.

In einem kg Vitamin-Mineral-Mischung sind enthalten:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x 5 $H_2O$ in Getreidemehl als Trägerstoff.

1 kg Wirkstoff-Prämix enthalten 100 g Wirkstoff, 900 g Weizenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung (Zusammensetzung wie beim Kükenfutter) 30 g Leinkuchenmehl, 30 g Maiskleberfutter, 10 g Sojaöl, 10 g Zuckerrohrmelasse und 2 g Wirkstoff-Prämix ergeben nach sorgfältigem Mischen 1 kg Futter.
1 kg Wirkstoff-Prämix enthält 200 g Wirkstoff, 20 g Pflanzenöl, 780 g Calciumcarbonatpulver.

Beispiel für die Zusammensetzung eines Futters für Rinder, das den erfindungsgemäßen Wirkstoff enthält:

69,95 % Futtergetreideschrot, 10 % gemahlene Maiskolben, 8 % Sojabohnenmehl, 5 % Luzernemehl, 5 % Melasse, 0,6 % Harnstoff, 0,5 % Calciumphosphat, 0,5 % Calciumcarbonat, 0,3 % Kochsalz, 0,15 % Vitamin-Mineral-Mischung und 0,2 % Wirkstoff-Prämix der beim Schweineaufzuchtfutter angegebenen Zusammensetzung. Die Vitamin-Mineral-Mischung enthält pro kg 70.000 i.E. Vitamin A, 70.000 i:E. Vitamin $D_3$, 100 mg Vitamin E, 50 mg Mn $SO_4$ x $H_2O$, 30 mg Zn $SO_4$ x $7H_2O$ in Getreidemehl als Trägerstoff.

Der Wirkstoff-Prämix wird der Vitamin-Mineral-Mischung in der erforderlichen Menge beigemischt und diese anschließend sorgfältig mit den übrigen Bestandteilen vermischt,

Die Wirkstoffe zeigen eine schwache antibiotische Wirksamkeit gegen grampositive Keime. Sie eignen sich zur Therapie und Prophylaxe der Schweinedysenterie sowie Coccidiose bei Geflügel und Säugetieren.

Die biologische Wirkung und die Synthese der Wirkstoffe wird durch die folgenden Beispiele erläutert:

Beispiel A

In-vitro Wiederkäuerversuch

Einem Hammel, der pro Tag 550 g grob gemahlenes Schaf-Fertigfutter, 400 g Heu und 250 g Trockengrün Cobs erhielt, wurde Pansenflüssigkeit durch eine Pansenfistel entnommen, 2,5 ml Pansenflüssigkeit wurden unmittelbar nach der Gewinnung in einem mit Kohlendioxid begasten Reagenzröhrchen von 13 ml Volumen mit 150 mg fein gemahlenem Schaf-Fertigfutter, 7,5 ml Pufferlösung und 0,5 ml einer 5 % wäßrigen Ethanollösung mit bzw. ohne erfindungsgemäßen Wirkstoff, vermischt. Die Pufferlösung, welche vor Anfang des Versuchs mit Kohlendioxid gesättigt wurde, enthielt pro Liter Wasser: 4,61 g $Na_2HPO_4$, 12,25 g $NaHCO_3$, 0,59 g NaCl, 0,71 g KCl, 0,32 g $MgCl_2$, 0,13 g $CaCl_2$.

Die Reagenzröhrchen wurden verschlossen und bei 39 °C bebrütet. Nach 24-stündiger Bebrütung wurde 1,0 ml der Fermentationsflüssigkeit aus den Ansätzen entnommen und in ein Eppendorf-Gefäß pipettiert, das 0,2 ml 10 %ige Phosphorsäure (enthaltend 5,7 µmol 2-Methylvaleriansäure) enthielt. Die Proben wurden bei 11 000 g zentrifugiert und aus dem Überstand die Konzentrationen der flüchtigen Fettsäuren gaschromatographisch bestimmt.

Bei jedem Versuch wurde das Verhältnis Essigsäure zu Propionsäure errechnet. Der bei den Negativkontrollen (= kein Wirkstoff) erhaltene Wert wurde mit 100 angesetzt und die Abweichungen im Verhältnis dazu angegeben. Je mehr Propionsäure gebildet wurde, um so niedriger liegt der Zahlenwert des Verhältnisses Essigsäure zu Propionsäure und um so kleiner wird die Verhältniszahl im Vergleich zur Kontrolle (niedrige Verhältniszahl = verringertes Essigsäure/Propionsäure-Verhältnis = verbesserte Futterverwertung). Zusätzlich wurden die Konzentrationen der Gesamtfettsäuren im Vergleich zur Kontrolle (=100) angegeben.

Die Ergebnisse dieses in vitro-Testes sind in der nachstehenden Tabelle beschrieben.

T a b e l l e A

| Wirkstoff Bsp. Nr. | Wirkstoff-konzentration (mg/Ansatz) | Essigsäure-Propionsäure-Verhältnis | Gesamt-fett-säuren |
|---|---|---|---|
| ohne Wirkstoff | 0 | 100,0 | 100,0 |
| Olaquindox (Vergleichs-substanz) | 0,1 | 102,4 | 98,9 |
| | 0,5 | 118,5 | 94,7 |
| | 1,0 | 127,7 | 81,4 |
| 1 | 0,1 | 98,1 | 97,3 |
| | 0,5 | 78,5 | 99,5 |
| | 1,0 | 68,9 | 97,1 |

Tabelle A (Fortsetzung)

| Wirkstoff Bsp. Nr. | Wirkstoffkonzentration (mg/Ansatz) | Essigsäure-Propionsäure-Verhältnis | Gesamtfettsäuren |
|---|---|---|---|
| 3 | 0,1 | 98,2 | 96,5 |
| | 0,5 | 79,3 | 102,1 |
| | 1,0 | 66,6 | 98,2 |
| 22 | 0,1 | 98,8 | 99,2 |
| | 0,5 | 76,5 | 101,3 |
| | 1,0 | 64,3 | 97,5 |
| 11 | 0,1 | 96,3 | 101,4 |
| | 0,5 | 74,5 | 99,8 |
| | 1,0 | 66,5 | 97,6 |
| 12 | 0,1 | 97,3 | 103,1 |
| | 0,5 | 80,4 | 99,9 |
| | 1,0 | 66,6 | 98,7 |
| 13 | 0,1 | 95,8 | 97,6 |
| | 0,5 | 75,4 | 101,4 |
| | 1,0 | 67,6 | 98,5 |

Die obige Tabelle zeigt, daß das Verhältnis von Essigsäure zu Propionsäure von den erfindungsgemäßen Verbindungen wesentlich herabgesetzt wird, ohne die Konzentration der Gesamtfettsäuren zu beeinträchtigen. Dagegen bewirkt Zugabe von Olaquindox eine Verschlechterung der Fermentation.

Beispiel B

Wiederkäuer-Fütterungsversuch

Zwölf Schafe, die jeweils mit Pansenfisteln versehen waren, wurden in 4 Gruppen von jeweils 3 Tieren verteilt. Die Tiere erhielten über einen Zeitraum von 4 Wochen pro Tag 650 g grob gemahlenes Schaffertigfutter, 250 g Trockengrün Cobs und 400 g grob gehäckseltes Heu. Die erfindungsgemäße Verbindung wurde im Fertigfutter einer Gruppe ab dem 22. Tag beigemischt, so daß die tägliche Aufnahme des Wirkstoffes 0,23, 0,46 bzw. 0,69 g betrug. Das Fertigfutter der vierten Gruppe enthielt keinen Wirkstoff (Kontroll-Gruppe). Am 17., 19., 21., 23., 25. und 27. Tag des Versuches wurden Pansensaftproben entnommen und die Konzentrationen der flüchtigen Fettsäuren gaschromatografisch bestimmt.

Wie in der nachstehenden Tabelle zu erkennen ist, erhöhte die Verfütterung der erfindungsgemäßen Verbindung den Gehalt an Propionsäure, ohne die Summe der Fettsäuren zu beeinträchtigen.

Tabelle B

| Wirkstoff Bsp. Nr. | Propionsäure (mol %) | Gesamtfettsäuren (mmol/l) |
|---|---|---|
| ohne Wirkstoff | 16,9 | 95,3 |
| 1 (0,23 g/Tag) | 17,3 | 99,8 |
| 1 (0,46 g/Tag) | 20,1 | 95,2 |
| 1 (0,69 g/Tag) | 21,0 | 98,7 |

Beispiele

Beispiel 1

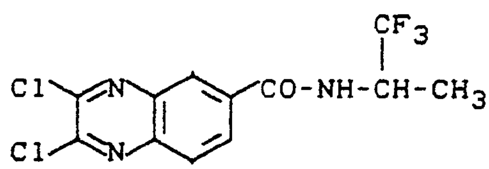

2,26 g (0,02 Mol) 1-Trifluormethyl-ethylamin und 2 g Pyridin werden in 100 ml wasserfreiem Acetonitril gelöst und dann unter Rühren 5,26 g (0,02 Mol) 2,3-Dichlorchinoxalin-6-carbonsäurechlorid gelöst in 20 ml Acetonitril zugetropft. Anschließend wird 3 Stunden auf 80° C erhitzt. Nach dem Abkühlen auf Raumtemperatur wird die Reaktionsmischung in 250 ml Wasser gegossen und 2 mal mit je 100 ml Ether extrahiert. Die Etherphase wird über Magnesiumsulfat getrocknet und anschließend der Ether im Vakuum abgezogen. Man erhält 3,5 g 2,3-Dichlorchinoxalin-6-carbonsäure-1-trifluormethyl-ethylamid als hellbraunes Pulver mit dem Schmp. 185° C.

Analog erhält man:

| Beispiel Nr. | | |
|---|---|---|
| 2 | 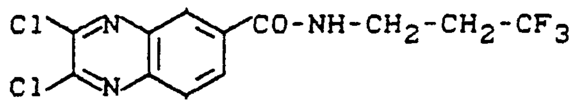 | Schmp.: 182° C |
| 3 | 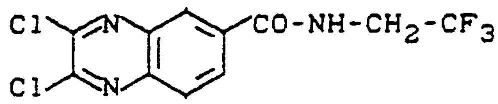 | Schmp.: 198° C |
| 4 | 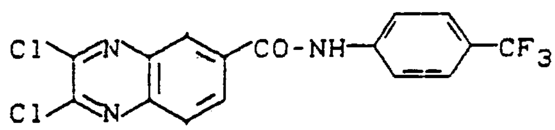 | Schmp.: 223° C |
| 5 | 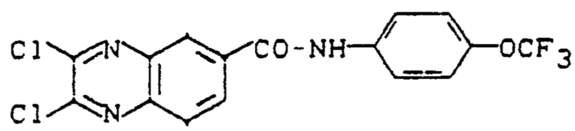 | Schmp.: 194° C |
| 6 | 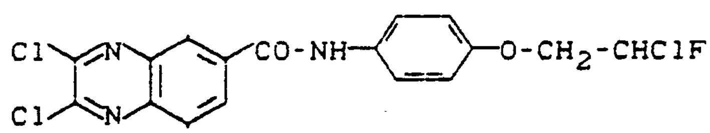 | Schmp.: 192° C |
| 7 | 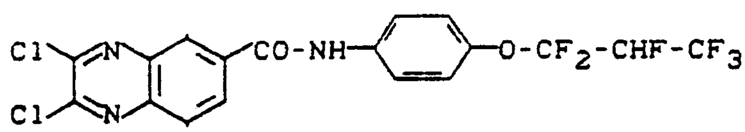 | Schmp.: 172° C |

Analog erhält man:

| Beispiel Nr. | | |
|---|---|---|
| 8 | 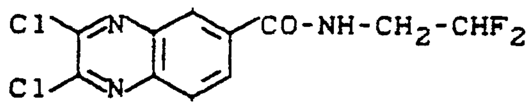 | |
| 9 | 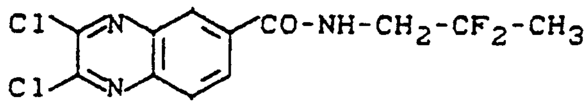 | |
| 10 | 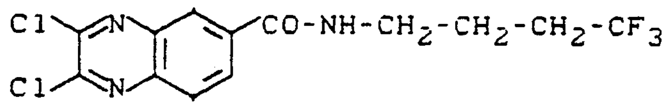 | |
| 11 | 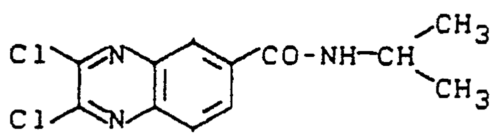 | Schmp.: 190° C |
| 12 | 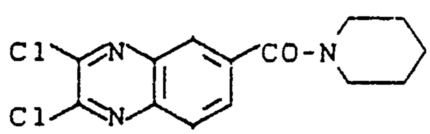 | Schmp.: 175° C |
| 13 | 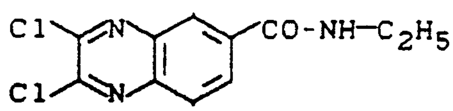 | Schmp.: 182° C |
| 14 | 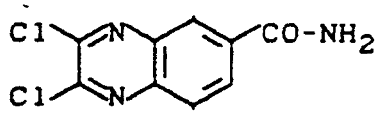 | |

Analog erhält man:

| Beispiel Nr. | |
|---|---|
| 14 | 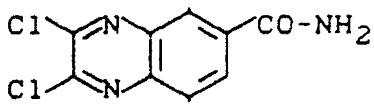 |
| 15 | 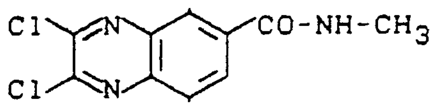 |
| 16 | 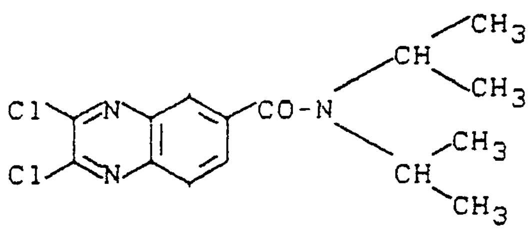 |
| 17 | 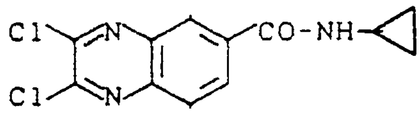 |
| 18 | 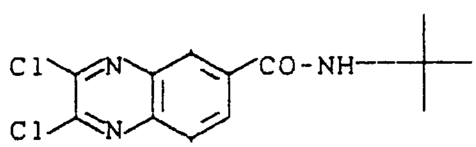 |
| 19 | 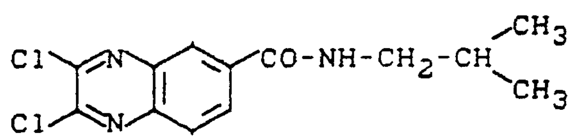 |
| 20 | 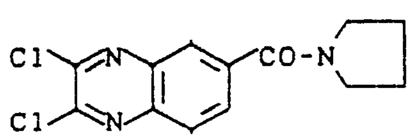 |

Analog erhält man unter Verwendung von 2,3-Dichlorchinoxalin-6-sulfonsäurechlorid folgende Verbindungen:

| Beispiel Nr. | | |
|---|---|---|
| 21 | 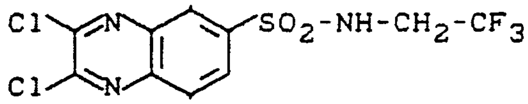 | Schmp.: 210 Zers. |
| 22 | 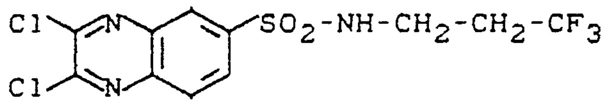 | Schmp. 224° C |
| 23 | 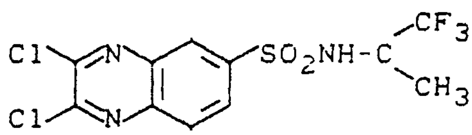 | |
| 24 | 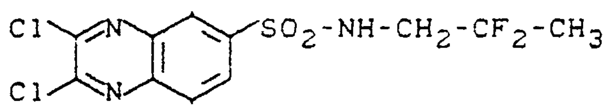 | |
| 25 | 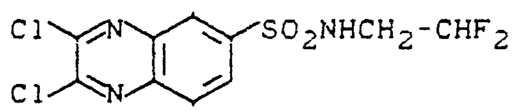 | |
| 26 | 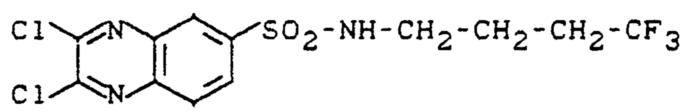 | |

**Patentansprüche**

**1.** Verwendung von 2,3-disubstituierten Chinoxalinen der Formel (I)

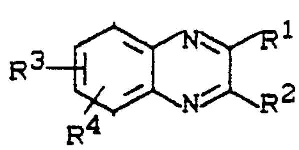

(I)

in welcher

| | |
|---|---|
| $R^1$ | für Halogen, OH, SH, Alkoxy, Aryloxy, Alkylthio, Arylthio steht, |
| $R^2$ | für Halogen, OH, SH, Alkoxy, Aryloxy, Alkylthio, Arylthio steht, |
| $R^1$ und $R^2$ | gemeinsam für den Rest der Formel |

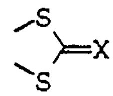

stehen, in welcher X für O oder S steht,

$R^3$ für Wasserstoff, Halogen, CN oder die Reste

$-CONR^5R^6$, $-SO_2NR^5R^6$, $-SO_2OR^5$,

CN
|
$-CH-R^7$, $-O-R^7$, $-COOR^5$ steht,

$R^4$ für H, Alkyl, Halogen, $NO_2$ oder den Rest $-CONR^5R^6$ steht,

$R^5$ für H, Alkyl oder Cycloalkyl steht, die gegebenenfalls substituiert sind oder Aryl steht, das gegebenenfalls substituiert ist,

$R^6$ für H, Alkyl oder Cycloalkyl steht, die gegebenenfalls substituiert sind oder für Aryl steht, das gegebenenfalls substituiert ist oder für den Rest $-S-R^7$ steht,

$R^5$ und $R^7$ können gemeinsam mit dem angrenzenden N-Atom einen gegebenenfalls substituierten heterocyclischen Ring bolden, der gegebenenfalls N- oder O- als weitere Heteroatome enthalten kann;

$R^7$ für gegebenenfalls substituiertes Alkyl oder Aryl steht,

zur Leistungsförderung von Tieren.

**2.** Mittel zur Leistungsförderung von Tieren, gekennzeichnet durch einen Gehalt an 2,3-disubstituierten Chinoxalinen der Formel (I) gemäß Anspruch 1.

**3.** Tierfütter, Trinkwasser für Tiere, Zusätze für Tierfutter und Trinkwasser für Tiere, gekennzeichnet durch einen Gehalt an 2,3-disubstituierten Chinoxalinen der Formel (I) gemäß Anspruch 1.

**4.** Verfahren zur Herstellung von Tierfutter, Trinkwasser für Tiere oder Zusätze für Tierfutter, Trinkwasser für Tiere, dadurch gekennzeichnet, daß man 2,3-disubstituierte Chinoxaline der Formel (I) gemäß Anspruch 1 mit Futtermitteln oder Trinkwasser und gegebenenfalls weiteren Hilfsstoffen vermischt.

**5.** Verwendung von 2,3-disubstituierten Chinoxalinen der Formel (I) gemäß Anspruch 1 zur Herstellung von Mitteln zur Leistungsförderung bei Tieren.

**6.** Neue 2,3-disubstituierte Chinoxaline der Formel (II)

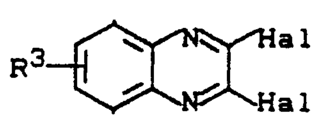

(II)

in welcher

$R^3$ für die Reste $-CONR^5R^6$ oder $-SO_2NR^5R^6$ steht,

$R^6$ für H, Alkyl steht,

$R^5$ für Alkyl steht das durch Fluor substituiert ist,

Hal für Chlor oder Brom steht.

**7.** Verfahren zur Herstellung der neuen 2,3-disubstituierten Chinoxaline der Formel (II)

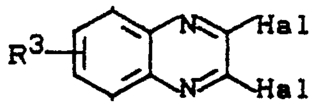

(II)

in welcher

$R^3$ für die Reste $-CONR^5R^6$ oder $-SO_2NR^5R^6$ steht,

$R^6$ für H, Alkyl steht,

$R^5$ für Alkyl steht das durch Fluor substituiert ist,

Hal für Chlor oder Brom steht,

dadurch gekennzeichnet, daß man 2,3-disubstituierte Chinoxaline der Formel (III)

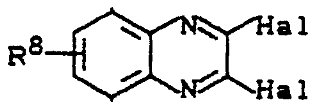

(III)

in welcher

$R^8$ für die Reste

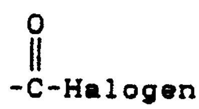

oder $-SO_2$-Halogen steht und

Hal für Chlor oder Brom steht,

mit Aminen der Formel (IV)

$H-NR^5R^6$ (IV)

in welcher

$R^5$ und $R^6$ die oben angegebene Bedeutung haben,

umsetzt.

Europäisches Patentamt

# EUROPÄISCHER RECHERCHENBERICHT

Nummer der Anmeldung

**EP 91 10 6891**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | DE-A-1 695 532 (MERCK & CO.INC)<br>* Seiten 1 - 7 & US-A-3510487 * | 1,2,6 | C 07 D 241/44<br>A 01 N 43/60 |
| A | GB-A-1 274 445 (IMPERIAL SMELTING CORPORATION (N.S.C.))<br>* das ganze Dokument * | 1,2,6 | |
| D,A | GB-A-1 178 930 (MERCK & CO.INC.)<br>* das ganze Dokument * | 1,2,6 | |
| A | US-A-3 141 886 (KLAUS SASSE ET AL.)<br>* das ganze Dokument * | 1,2,6 | |
| A | US-A-4 328 228 (JOHN B.ADAMS)<br>* das ganze Dokument * | 1,2,6 | |

RECHERCHIERTE SACHGEBIETE (Int. Cl.5)

C 07 D 241/00

Der vorliegende Recherchenbericht wurde für alle Patentansprüche erstellt

| Recherchenort | Abschlußdatum der Recherche | Prüfer |
|---|---|---|
| Berlin | 22 August 91 | KYRIAKAKOU G |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument